(19) Europäisches Patentamt
European Patent Office
Office européen des brevets

(11) EP 0 548 024 B1

(12) EUROPÄISCHE PATENTSCHRIFT

(45) Veröffentlichungstag und Bekanntmachung des Hinweises auf die Patenterteilung:
**08.01.1997 Patentblatt 1997/02**

(51) Int Cl.[6]: **C07K 5/06**, C07C 323/60, A61K 38/05

(21) Anmeldenummer: **92810989.1**

(22) Anmeldetag: **11.12.1992**

(54) **Aminosulfonsäurederivate und Verfahren zu ihrer Herstellung**

Aminosulphonic acid derivatives and process for their preparation

Dérivés d'acides aminosulfoniques et procédé pour leur préparation

(84) Benannte Vertragsstaaten:
**AT BE CH DE DK ES FR GB GR IE IT LI LU NL PT SE**

(30) Priorität: **19.12.1991 CH 3776/91**

(43) Veröffentlichungstag der Anmeldung:
**23.06.1993 Patentblatt 1993/25**

(73) Patentinhaber: **CIBA-GEIGY AG**
**4002 Basel (CH)**

(72) Erfinder:
- **Baschang, Gerhard, Dr.**
**CH-4126 Bettingen (CH)**
- **Hartmann, Albert, Dr.**
**W-7889 Grenzach (DE)**

(56) Entgegenhaltungen:
**EP-A- 0 000 330** **EP-A- 0 114 787**

- **INTERNATIONAL JOURNAL OF PEPTIDE AND PROTEIN RESEARCH Bd. 37, Nr. 1 , Januar 1991 , COPENHAGEN DK Seiten 46 - 57 J. METZGER ET AL. 'Synthesis of novel immunologically active tripalmitoyl-S-glycerylcysteinyl lipopeptides as useful intermediates for immunogen preparations'**

EP 0 548 024 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die Erfindung betrifft N-acylierte Derivate einer unsubstituierten oder durch Carboxy substituierten ω-Amino-$C_2$-$C_3$-alkansulfonsäure, Verfahren zu ihrer Herstellung und ihre Verwendung zur Herstellung pharmazeutischer Präparate und als Arzneimittel, insbesondere mit immunstimulierender Wirkung.

Insbesondere betrifft die Erfindung Salze von Aminosulfonsäurederivaten der Formel I,

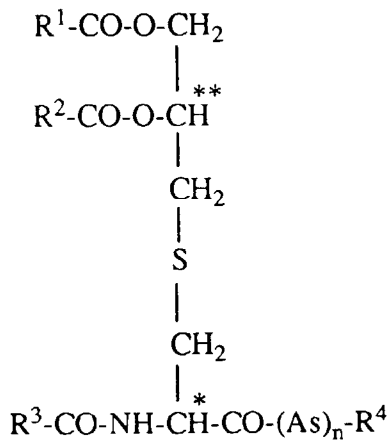

* (R)-Konfiguration

** (R)- oder (S)-Konfiguration

(I)

worin $R^1$, $R^2$ und $R^3$ unabhängig voneinander je einen aliphatischen Kohlenwasserstoffrest mit 7 bis 21 C-Atomen bedeuten, n für 0 oder 1 steht, As den amidisch gebundenen Rest einer (D)- oder (L)-Aminosäure oder eines (D)- oder (L)-Aminosäurederivats aus der Gruppe, bestehend aus Gly, Ala, Ser, Thr, Asp, Asp($R^5$), Glu, Glu($R^5$), Gla, Gla($R^5$) und Gla$(R^5)_2$ bedeutet und $R^4$ und $R^5$ unabhängig voneinander für den amidisch gebundenen Rest einer unsubstituierten oder durch Carboxy substituierten ω-Amino-$C_2$-$C_3$-alkansulfonsäure stehen.

Salzbildende Gruppen in einer Verbindung der Formel I sind die Sulfonsäuregruppen in den Resten $R^4$ und $R^5$ sowie freie Carboxylgruppen in den Resten As, $R^4$ und $R^5$. Salze einer Verbindung der Formel I sind Metall- oder Ammoniumsalze und sind vorzugsweise pharmazeutisch-verwendbar und nicht-toxisch, z.B. Alkalimetall- oder Erdalkalimetallsalze, z.B. Natrium-, Kalium-, Magnesium- oder Calciumsalze, oder Salze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triethylamin, Hydroxyniederalkylamine, z.B. 2-Hydroxyethylamin, Bis-(2-hydroxyethyl)-amin, 2-Hydroxy-ethyl-diethyl-amin oder Tri-(2-hydroxyethyl)-amin, basische aliphatische Ester von Carbonsäuren, z.B. 4-Aminobenzoesäure-2-diethylaminoethylester, Niederalkylenamine, z.B. 1-Ethylpiperidin, Cycloalkylamine, z.B. Dicyclohexylamin, oder Benzylamine, z.B. N,N'-Dibenzylethylendiamin, ferner Basen vom Pyridintyp, z.B. Pyridin, Collidin oder Chinolin. Bei Anwesenheit von mehreren sauren Gruppen können Mono- oder Polysalze gebildet werden.

Zur Isolierung oder Reinigung können auch pharmazeutisch ungeeignete Salze Verwendung finden. Zur therapeutischen Anwendung gelangen jedoch nur die pharmazeutisch verwendbaren, nicht-toxischen Salze, die deshalb bevorzugt werden.

Ein aliphatischer Kohlenwasserstoffrest $R^1$, $R^2$ oder $R^3$ ist vorzugsweise $C_7$-$C_{21}$-Alkyl, $C_{17}$-Alkenyl oder $C_{17}$-Alkinyl. $C_7$-$C_{21}$-Alkyl $R^1$, $R^2$ oder $R^3$ ist vorzugsweise geradkettig, z.B. n-Heptyl, n-Octyl, n-Nonyl, n-Decyl, n-Undecyl, n-Tridecyl, n-Pentadecyl, n-Hexadecyl, n-Heptadecyl, n-Nonadecyl oder n-Heneicosyl. Von den genannten Alkylresten sind diejenigen mit 11 - 17 C-Atomen besonders bevorzugt. $C_{17}$-Alkenyl $R^1$, $R^2$ oder $R^3$ ist vorzugsweise (Z)-Heptadec-9-en-1-yl, (E)-Heptadec-9-en-1-yl, Heptadeca-6(Z),9(Z)-dienyl oder Heptadecatrienyl. $C_{18}$-Alkinyl ist vorzugsweise Heptadec-9-in-1-yl. Somit leiten sich die Acylreste $R^1$-CO-, $R^2$-CO- und $R^3$-CO- unabhängig voneinander vorzugsweise von einer $C_8$-$C_{22}$-Alkansäure, einer $C_{18}$-Alkensäure oder einer $C_{18}$-Alkinsäure ab. Eine $C_8$-$C_{22}$-Alkansäure ist insbesondere z.B. Caprylsäure (n-Octansäure), Pelargonsäure (n-Nonansäure), Caprinsäure (n-Decansäure), Undecylsäure (n-Undecansäure), Laurinsäure, Myristinsäure, Palmitinsäure, Margarinsäure, Stearinsäure, Arachinsäure (n-Eicosansäure) oder Behensäure (n-Docosansäure). Eine $C_{18}$-Alkensäure ist insbesondere z.B. Oelsäure, Elaidinsäure, Linolsäure, α-Linolensäure oder α- oder β-Eläostearinsäure.

Eine $C_{18}$-Alkinsäure ist insbesondere z.B. Stearolsäure.

$R^1$ und $R^2$ können verschiedene Bedeutungen oder vorzugsweise die gleiche Bedeutung haben.

Die Abkürzungen Gly, Ala, Ser, Thr, Asp, Glu oder Gla bezeichnen in dieser Reihenfolge die Aminosäuren Glycin,

Alanin, Serin, Threonin, Asparaginsäure, Glutaminsäure bzw. γ-Carboxy-Glutaminsäure.

In Uebereinstimmung mit den international anerkannten Nomenklaturregeln bezeichnen in dieser Anmeldung die Abkürzungen für die Aminosäuren die freie Säure und, wenn nicht anders angegeben, die L-Konfiguration. Die α-Aminogruppe ist an der linken Seite der Abkürzung, die Carboxygruppe an der rechten Seite zu denken. Das Fehlen eines H-Atoms in der α-Amino-Gruppe wird durch einen links an der Abkürzung für die Aminosäure stehenden Bindestrich, das Fehlen von zwei H-Atomen durch zwei links stehende Bindestriche gekennzeichnet. Das Fehlen einer HO-Gruppe in der Carboxy-gruppe wird durch einen rechts stehenden Bindestrich ausgedrückt. Substituenten in der Seitenkette von Aminosäuren werden unmittelbar hinter das Aminosäuresymbol in Klammern gesetzt. Somit steht z.B. Palmitoyl-Cys[2(R,S),3-dilauroyloxy-propyl] für N-Palmitoyl-S-[2(R,S),3-di-lauroyloxy-propyl]-(R)-cystein.

Eine ω-Amino-$C_2$-$C_3$-alkansulfonsäure ist z.B. Homotaurin der Formel $H_2N-(CH_2)_3-SO_3H$ oder vorzugsweise Taurin der Formel $H_2N-(CH_2)_2-SO_3H$. Eine durch Carboxy substituierte ω-Amino-$C_2$-$C_3$-alkansulfonsäure ist vorzugsweise Cysteinsäure der Formel $H_2N-CH(COOH)-CH_2-SO_3H$, insbesondere in der (L)-Konfiguration.

Die Salze der Sulfonsäurederivate der Formel I besitzen wertvolle pharmakologische, insbesondere immunstimulierende Eigenschaften.

So sind diese Salze in der Lage, bereits in einer Dosierung von 0,02 Nanogramm/0,2 ml Kulturmedium tumorizide Ratten-Alveolarmakrophagen in vitro zu aktivieren, so dass diese nach Inkubation mit dem Salz Tumorzellen abtöten können.

Diese Salze induzieren in vitro auch die Freisetzung von Interleukin-1β und Tumornekrose -Faktor (TNFα) aus menschlichen Monozyten.

Auch in in vivo Modellen zeichnen sich die neuen Verbindungen durch hohe biologische Aktivität aus: NMRI Mäuse werden durch intraperitoneale Injektion von 10 μg bovinem Serumalbumin (BSA) an Tag 0 immunisiert. 8, 18 und 28 Tage später werden Serumproben entnommen und auf ihren Gehalt an anti-BSA Antikörpern mit einer passiven Haemagglutinationstechnnik untersucht. In der verwendeten Dosis ist das BSA für die Empfängertiere subimmunogen, d.h. es vermag keine oder nur eine geringfügige Produktion von Antikörpern auszulösen. Im diesem Test sind nun die Salze der Verbindungen der Formel I in der Lage, bei intraperitonealer Applikation am Tage der Immunisierung die Antikörperproduktion gegen BSA signifikant zu steigern. Besonders hervorzuheben ist die gute Wirksamkeit der neuen Verbindungen bei s.c. Applikation.

Im Gegensatz zu C57BL/6 Mäusen sind MAGf Mäuse anfällig für eine Infektion durch L. monocytogenes. MAGf Mäuse werden 72 und 24 Stunden vor der Infektion peroral mit einer Suspension von 1 mg/kg einer Verbindung der Formel I in Squalen oder einem äquivalenten Volumen Squalen behandelt. Danach werden die Mäuse intravenös mit 1 x $10^4$ CFU (colony forming units) L. monocytogenes EX 1 infiziert. 10 Tage nach der Infektion wird testgestellt, dass die Ueberlebensrate in der Gruppe der mit einer Verbindung der Formel I behandelten MAGf Mäuse signifikant höher ist, z.B. 80 %, als in der Gruppe der mit einen äquivalenten Volumen Squalen behandelten Mäuse (20 %). Die Ueberlebensrate von mit Squalen behandelten C57BL/6 Mäusen betragt 90 %.

12-15 Monate alte C57BL/6 Mäuse sind anfällig für eine Infektion mit H. influenzae, während 6-8 Wochen junge Mäuse resistent sind. 72 und 24 Stunden vor der Infektion werden die Mäuse peroral mit einer Suspension von 1 mg/kg einer Verbindung der Formel I in Squalen oder einem äquivalenten Volumen Squalen behandelt. Danach werden die Mäuse intravenös mit 1 x $10^5$ CFU (colony forming units) H. influenzae P27 infiziert. 10 Tage nach der Infektion wird festgestellt, dass die Ueberlebensrate in der Gruppe der mit einer Verbindung der Formel I behandelten alten Mäuse signifikant höher ist, z.B. 70 %, als in der Gruppe der mit einem äquivalenten Volumen Squalen behandelten Mäuse (10 %). Von den mit Squalen behandelten jungen Mäusen überleben 80 %.

Die neuen Salze der Verbindungen der Formel I können als Adjuvantien in Mischung mit Impfstoffen oder chemisch gebunden an Impfstoffe, z.B. geeignete Antigene oder Haptene, dazu benützt werden, den Impferfolg zu verbessern und den durch humorale Antikörper und/oder zelluläre Immunität vermittelten Infektschutz gegenüber bakteriellen, viralen oder parasitären Erregern zu verbessern.

Die neuen Salze können dazu benützt werden, Immunreaktionen bei Mensch und Tier zu fördern. Die Salze eignen sich demnach besonders für die Stimulation der körpereigenen Abwehr, z.B. bei Krebs, chronischen und akuten Infektionen oder bei selektiven (antigenspezifischen) immunologischen Defekten, sowie bei angeborenen, aber auch bei erworbenen allgemeinen (d.h. nicht antigenspezifischen) immunologischen Defektzuständen, wie sie im Alter, im Verlauf schwerer Primärerkrankungen und vor allem nach Therapie mit ionisierenden Strahlen oder mit immunosuppressiv wirkenden Pharmaka auftreten. Die genannten Verbindungen können auch in Kombination mit Antibiotika, Chemotherapeutika oder anderen Stoffen verabreicht werden, um immunologischen Schädigungen entgegenzuwirken. Schliesslich sind die beschriebenen Verbindungen auch zur allgemeinen Prophylaxe von Infektionskrankheiten bei Mensch und Tier geeignet.

Die Dosierung des Wirkstoffes hängt u.a. von der Warmblüterspezies, der Abwehrlage des Organismus, der Applikationsweise und der Art der Erkrankung ab. Die Dosis-Wirkungsbeziehung ist relativ schwach ausgeprägt.

Die therapeutische Dosis für Warmblüter von etwa 70 kg Körpergewicht, z.B. den Menschen, liegt zwischen 0,1 mg und 25 mg pro Tag, vorzugsweise zwischen 0,2 und 10 mg, hauptsächlich zwischen 0,5 mg und 5 mg, z.B. bei 2

mg, z.B. bei intravenöser Applikation. Die Dosierung bei topischer Applikation liegt bis zum Faktor 10 niedriger.

Bevorzugt sind die Salze von Sulfonsäurederivaten der Formel I, worin $R^1$, $R^2$ und $R^3$ unabhängig voneinander $C_7$-$C_{21}$-Alkyl, $C_{17}$-Alkenyl oder $C_{17}$-Alkinyl bedeuten, n für 0 oder 1 steht, As den amidisch gebundenen Rest einer Aminosäure oder eines Aminosäurederivats aus der Gruppe, bestehend aus Ala und Glu($R^5$) bedeutet, und $R^4$ und $R^5$ je für den amidisch gebundenen Rest einer ω-Amino-$C_2$-$C_3$-alkansulfonsäure stehen.

Besonders bevorzugt sind die Salze von Sulfonsäurederivaten der Formel I, worin $R^1$ und $R^2$ jeweils die gleiche Bedeutung haben und jeweils für geradkettiges $C_{11}$-$C_{17}$-Alkyl stehen, $R^3$ geradkettiges $C_{11}$-$C_{17}$-Alkyl bedeutet, n für 0 oder 1 steht, As den amidisch gebundenen Rest von Alanin oder Glu(NH-$CH_2$-$CH_2$-$SO_3H$) bedeutet, und $R^4$ für den amidisch gebundenen Rest von 2-Amino-ethansulfonsäure steht.

Ganz besonders bevorzugt sind die obengenannten Salze, worin in Formel I $R^1$ für n-Undecyl, $R^2$ für n-Undecyl und $R^3$ für n-Pentadecyl stehen.

Vor allem sind die in den Beispielen beschriebenen Salze der Verbindung der Formel I bevorzugt.

Die Salze der Verbindungen der Formel I werden in an sich bekannter Weise hergestellt.

Sie werden z.B. hergestellt, indem man

a) eine Verbindung der Formel II,

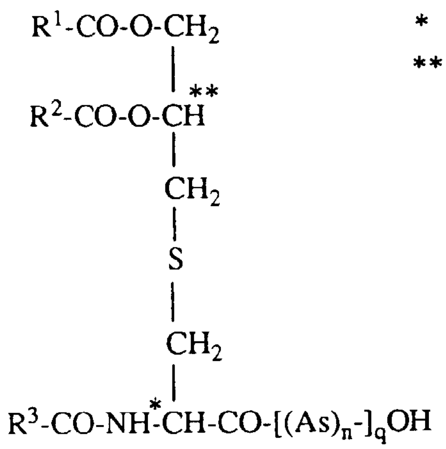

* (R)-Konfiguration

** (R)- oder (S)-Konfiguration

(II)

worin q für 0 oder 1 steht und $R^1$, $R^2$, $R^3$, n und As die obengenannten Bedeutungen haben, wobei im Rest As vorhandene freie funktionelle Gruppen mit Ausnahme der Gruppe, die an der Reaktion teilnehmen soll, erforderlichenfalls durch leicht abspaltbare Schutzgruppen geschützt sind, oder ein reaktionsfähiges Carbonsäurederivat davon mit dem Salz einer Verbindung der Formel III,

$$H\text{-}[(As)_n\text{-}]_r R^4 \qquad \text{(III)}$$

worin r für 1 steht, wenn im Reaktionspartner der Formel II q für 0 steht, oder r für 0 steht, wenn q für 1 steht, und worin As, n und $R^4$ die obengenannten Bedeutungen haben, wobei in den Resten As und $R^4$ vorhandene freie funktionelle Gruppen mit Ausnahme der Gruppe, die an der Reaktion teilnehmen soll, erforderlichenfalls durch leicht abspaltbare Schutzgruppen geschützt sind, umsetzt und vorhandene Schutzgruppen abspaltet, oder

b) ein Salz einer Verbindung der Formel IV,

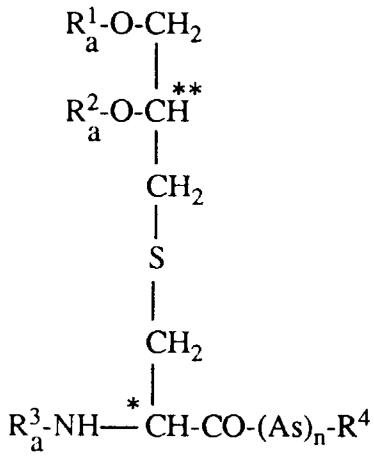

* (R)-Konfiguration

** (R)- oder (S)-Konfiguration

(IV)

worin $R^1a$ für Wasserstoff oder den obengenannten Rest $R^1$-CO-, $R^2a$ für Wasserstoff oder den obengenanten Rest $R^2$-CO- und $R^3a$ für Wasserstoff oder den obengenannten Rest $R^3$-CO- stehen, wobei mindestens einer der Reste $R^1a$, $R^2a$ und $R^3a$ Wasserstoff bedeuten muss, und worin As, n und $R^4$ die obengenannten Bedeutungen haben, wobei in den Resten As und $R^4$ vorhandene freie funktionelle Gruppen mit Ausnahme der Gruppe, die an der Reaktion teilnehmen soll, erforderlichenfalls durch leicht abspaltbare Schutzgruppen geschützt sind, mit einer Carbonsäure der Formel V,

$$R^6\text{-COOH} \quad (V)$$

worin $R^6$ einen aliphatischen Kohlenwasserstoffrest mit 7 bis 21 C-Atomen bedeutet, oder mit einem reaktionsfähigen Carbonsäurederivat davon umsetzt und vorhandene Schutzgruppen abspaltet, oder
c) eine Verbindung der Formel VI,

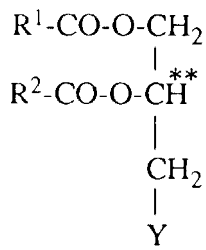

** (R)- or (S)-Konfiguratior

(VI)

worin Y für eine nucleofuge Gruppe steht und $R^1$ und $R^2$ die obengenannten Bedeutungen haben, mit dem Salz einer Verbindung der Formel VII,

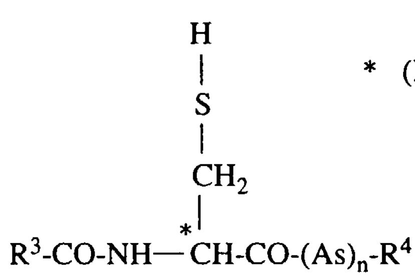

* (R)-Konfiguration

(VII)

worin die Substituenten die obengenannten Bedeutungen haben, wobei freie funktionelle Gruppen mit Ausnahme der an der Reaktion teilnehmenden Mercaptogruppe, wenn nötig, durch leicht abspaltbare Schutzgruppen geschützt sind, oder mit einem reaktionsfähigen Derivat einer Verbindung der Formel VII umsetzt und vorhandene Schutzgruppen abspaltet, oder

(d) eine Verbindung der Formel VIII,

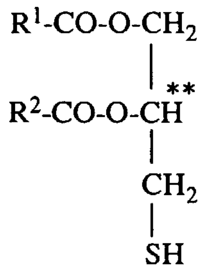

** (R)- or (S)-Konfiguration

(VIII)

worin $R^1$ und $R^2$ die obengenannten Bedeutungen haben, oder ein reaktionsfähiges Derivat dieser Verbindung mit dem Salz einer Verbindung der Formel IX,

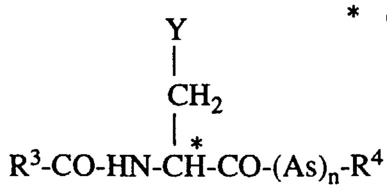

* (R)-Konfiguratior

(IX)

worin Y für eine nucleofuge Gruppe steht und die übrigen Substituenten die obengenannten Bedeutungen haben, wobei freie funktionelle Gruppen, wenn nötig, in geschützter Form vorliegen, umsetzt und vorhandene Schutzgruppen abspaltet, und, wenn erwünscht, nach Durchführung einer der Verfahrensvarianten a - d) ein erhaltenes Salz in ein anderes Salz überführt und, wenn erwünscht, erhaltene Isomerengemische auftrennt.

Die Durchführung der obengenannten Verfahrensvarianten wird im folgenden näher erläutert:

Bevorzugt sind die Verfahren b) und insbesondere a).

Verfahren a): In einer Verbindung der Formeln II und III gegebenenfalls vorhandene freie funktionelle Gruppen, die durch leicht abspaltbare Schutzgruppen geschützt werden, sind insbesondere freie Carboxyl-Gruppen, die nicht acyliert werden sollen. Fakultativ, d.h. nicht unbedingt erforderlich, ist der Schutz von freiem Hydroxy im Rest As.

Schutzgruppen, ihre Einführung und Abspaltung sind beispielsweise beschrieben in "Protective Groups in Organic Chemistry", Plenum Press, London, New York 1973,und in "Methoden der organischen Chemie", Houben-Weyl, 4. Auflage, Bd. 15/1, Georg-Thieme-Verlag, Stuttgart 1974 sowie in Theodora W. Greene, "Protective Groups in Organic Synthesis, John Wiley & Sons, New York 1981. Charakteristisch für Schutzgruppen ist, dass sie leicht, d.h. ohne dass unerwünschte Nebenreaktionen stattfinden, z.B. solvolytisch, reduktiv, photolytisch oder auch unter physiologischen Bedingungen abspaltbar sind.

Hydroxyschutzgruppen sind z.B. Acylreste, wie gegebenenfalls, z.B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z.B. 4-Nitrobenzyloxycarbonyl, oder Diphenylmethoxycarbonyl, oder 2-Halogen-niederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z.B. tert.-Butyl, 2-oxa- oder 2-thia-aliphatische oder -cycloaliphatische Kohlenwasserstoffreste, in erster Linie 1-Niederalkoxyniederalkyl oder 1-Niederalkylthioniederalkyl, z.B. Methoxymethyl, 1-Methoxy-ethyl, 1-Ethoxy-ethyl, Methylthiomethyl, 1-Methylthioethyl oder 1-Ethylthioethyl, oder 2-Oxa- oder 2-Thiacycloalkyl mit 5-6 Ringatomen, z.B. Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Carboxylgruppen sind üblicherweise in veresterter Form geschützt, wobei solche Estergruppierungen unter schonenden Bedingungen leicht spaltbar sind. In dieser Art geschützte Carboxylgruppen enthalten als veresternde Gruppen

in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, Arylmethoxycarbonyl mit einem oder zwei Arylresten, wobei diese gegebenenfalls z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls, z.B. wie oben erwähnt, substituiertes Benzyloxycarbonyl, z.B. 4-Methoxybenzyloxycarbonyl, oder 4-Nitrobenzyloxycarbonyl, oder gegebenenfalls, z.B. wie oben erwähnt, substituiertes Diphenylmethoxycarbonyl, z. B. Diphenylmethoxycarbonyl oder Di-(4-methoxyphenyl)methoxycarbonyl, 1-Niederalkoxyniederalkoxycarbonyl, wie Methoxymethoxycarbonyl, 1 -Methoxyethoxycarbonyl oder 1-Ethoxymethoxycarbonyl, 1-Niederalkylthioniederalkoxycarbonyl, wie 1-Methylthiomethoxycarbonyl oder 1-Ethylthioethoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)ethoxycarbonyl, worin die Substituenten unabhangig voneinander je einen gegebenenfalls substituierten, z.B. durch Niederalkyl, Niederalkoxy, Aryl, Halogen, und/oder Nitro substituierten, aliphatischen, araliphatischen, cycloalaphatischen oder aromatischen Kohlenwasserstoffrest, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, 2-Trimethylsilylethoxycarbonyl oder 2-(Di-n-butylmethyl-silyl)-ethoxycarbonyl, oder 2-Triarylsilylethoxycarbonyl, wie 2-Triphenylsilylethoxycarbonyl.

Die oben und im folgenden erwähnten organischen Silyl- oder Stannylreste enthalten vorzugsweise Niederalkyl, insbesondere Methyl, als Substituenten der Silizium- oder Zinn-Atome. Entsprechende Silyl- oder Stannylgruppen sind in erster Linie Triniederalkylsilyl, insbesondere Trimethylsilyl, ferner Dimethyl-tert.-butyl-silyl, oder entsprechend substituiertes Stannyl, z.B. Tri-n-butylstannyl.

Bevorzugte geschützte Carboxylgruppen sind tert.-Niederalkoxycarbonyl, wie tert.-Butoxycarbonyl, und in erster Linie gegebenenfalls, z.B. wie oben erwähnt, substituiertes Benzyloxycarbonyl, wie 4-Nitrobenzyloxycarbonyl, oder Diphenylmethoxycarbonyl, vor allem 2-(Trimethylsilyl)ethoxycarbonyl.

Als Acylierungsmittel zur Acylierung einer Verbindung der Formel III verwendbare reaktionsfähige Carbonsäurederivate einer Verbindung der Formel II sind in erster Linie reaktionsfähige aktivierte Ester oder reaktionsfähige Anhydride, ferner reaktionsfähige cyclische Amide, wobei die Aktivierung der als Acylierungsmittel verwendeten Carbonsäure auch in situ in Gegenwart der Verbindung der Formel III erfolgen kann.

Aktivierte Ester von Säuren sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch Behandeln der entsprechenden Säure mit einem Isoxazoliumreagens erhalten kann; 1,2-Oxazolium- oder Woodward-Methode), oder I-Niederalkoxyvinylester (die man z.B. durch Behandeln der entsprechenden Säure mit einem Niederalkoxyacetylen erhalten kann; Ethoxyacetylen-Methode), oder Ester vom Amidinotyp, wie N,N'-disubstituierte Amidinoester (die man z.B. durch Behandeln der entsprechenden Säure mit einem geeigneten N,N'-disubstituierten Carbodiimid, z.B. N,N'-Dicyclohexylcarbodiimid, erhalten kann; Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode), geeignete Arylester, insbesondere durch Elektronenanziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlorphenol oder 4-Phenyldiazophenol, in Gegenwart eines Kondensationsmittels, wie N,N'-Dicyclohexylcarbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere gegebenenfalls, z.B. duch Nitro, substituierte Phenyl-thioester (die man z.B. durch Behandeln der entsprechenden Säure mit gegebenenfalls, z. B. durch Nitro, substituierten Thiophenolen, u.a. mit Hilfe der Anhydrid- oder Carbo-diimid-Methode, erhalten kann; Methode der aktivierten Thiolester), Amino-oder Amidoester (die man z.B. durch Behandeln der entsprechenden Säure mit einer N-Hydroxy-amino- bzw. N-Hydroxy-amido-Verbindung, z.B. N-Hydroxy-succinimid, N-Hydroxy-piperidin, N-Hydroxy-phthalimid oder 1-Hydroxy-benztriazol, z.B. nach der Anhydrid- oder Carbodiimid-Methode, erhalten kann; Methode der aktivierten N-Hydroxyester) oder Silylester (die man z.B. durch Behandeln der entsprechenden Säure mit einem Silylierungsmittel, z.B. Hexamethyldisilazan, erhalten kann und die leicht mit Hydroxy-, nicht aber mit Aminogruppen reagieren).

Anhydride von Sauren können symmetrische oder vorzugsweise gemischte Anhydride dieser Säuren sein, so z. B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechloridmethode), Azide (die man z.B. aus einem entsprechenden Säureester über das entsprechende Hydrazid und dessen Behandlung mit salpetriger Säure erhalten kann; Azidmethode), Anhydride mit Kohlensäurehalbderivaten, wie mit entsprechenden Estern, z.B. Kohlensäureniederalkylhalbestern (die man z.B. durch Behandeln der entsprechenden Säure mit Halogen-, wie Chlorameisensäure-niederalkylestern oder mit einem 1 -Niederalkoxycarbonyl-2-niederalkoxy-1,2-dihydro-chinolin, z.B. 1-Niederalkoxycarbonyl-2-ethoxy-1,2-dihydrochinolin, erhalten kann; Methode der gemischten O-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychloridmethode), oder Anhydride mit organischen Säuren, wie gemischte Anhydride mit organischen Carbonsäuren (die man z.B. durch Behandeln der entsprechenden Säure mit einem gegebenenfalls substituierten Niederalkan- oder Phenylalkancarbonsäurehalogenid, z.B. Phenylessigsäure-, Pivalinsäure- oder Trifluoressigsäurechlorid erhalten kann; Methode der gemischten Carbonsäureanhydride) oder mit organischen Sulfonsäuren (die man z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N'-Carbonyldiimidazol erhalten kann; Imidazolid-Methode), oder Pyrazolen, z.B. 3,5-Dimethyl-pyrazol (die man z.B. über das Säurehydrazid durch Behandeln mit Acetylaceton erhalten kann; Pyrazolid-Methode).

Wie erwähnt können Derivate von Säuren, die als Acylierungsmittel verwendet werden, auch in situ gebildet werden. So kann man z.B. N,N'-disubstituierte Amidinoester in situ bilden, indem man das Gemisch des Ausgangsmaterials der Formel III und der als Acylierungsmittel verwendeten Säure in Gegenwart eines geeigneten N,N-disubstituierten Carbodiimids, z.B. N,N'-Dicyclohexylcarbodiimid, zur Reaktion bringt. Ferner kann man Amino- oder Amidoester der als Acylierungsmittel verwendeten Säuren in Gegenwart des zu acylierenden Ausgangsmaterials der Formel III bilden, indem man das Gemisch der entsprechenden Säure- und Amino-Ausgangsstoffe in Gegenwart eines N,N'-disubstituierten Carbodiimids, z.B. N,N'-Dicyclohexyl-carbodimid, und eines N-Hydroxy-amins oder N-Hydroxyamids, z.B. N-Hydroxysuccinimid, N-Hydroxy-norbornan-2,3-dicarboximid oder N-Hydroxy-benztriazol, gegebenenfalls in Anwesenheit einer geeigneten Base, z.B. 4-Dimethylamino-pyridin oder Tetramethylguanidin, umsetzt.

Vorzugsweise verwendet man die Verbindungen der Formel III in Form ihrer Alkalimetall - wie insbesondere Natriumsalze.

Die Reaktion kann in an sich bekannter Weise durchgeführt werden, wobei die Reaktionsbedingungen in erster Linie davon abhängen, ob und wie die Carboxylgruppe des Acylierungsmittels aktiviert ist, üblicherweise in Gegenwart eines geeigneten Lösungs- oder Verdünnungsmittels oder eines Gemisches von solchen, und, falls notwendig, in Gegenwart eines Kondensationsmittels, das z.B., wenn die an der Reaktion teilnehmende Carboxylgruppe als Anhydrid vorliegt, auch ein Säure-bindendes Mittel sein kann, unter Kühlen oder Erwärmen, z.B. in einem Temperaturbereich von etwa -30°C bis etwa +150°C, insbesondere von etwa 0°C bis +70°C, vorzugsweise von Raumtemperatur (ca. +20°C) bis +40°C, in einem geschlossenen Reaktionsgefäss und/oder in der Atmosphäre eines Inertgases, z.B. Stickstoff. Uebliche Kondensationsmittel sind z.B. Carbodiimide, beispielsweise N,N'-Diethyl-, N,N'-Dipropyl-, N,N'-Dicyclohexyl- oder N-Ethyl-N'-(3-dimethylaminopropyl)-carbodiimid, geeignete Carbonylverbindungen, beispielsweise Carbonyldiimidazol, oder 1,2-Oxazoliumverbindungen, z.B. 2-Ethyl-5-phenyl-1,2-Oxazolium-3'-sulfonat und 2-tert.-Butyl-5-methylisoxazolium-perchlorat, oder eine geeignete Acylaminoverbindung, z.B. 2-Ethoxy-1-ethoxycarbonyl-1,2-dihydrochinolin. Uebliche säurebindende Kondensationsmittel sind z.B. Alkalimetallcarbonate oder -hydrogencarbonate, z.B. Natrium- oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-ethyl-amin.

Die Abspaltung der Schutzgruppen, z.B. der Carboxyl-, Amino-, Hydroxy- oder Carbamoylschutzgruppen, die nicht Bestandteil des gewünschten Endprodukts der Formel I sind, erfolgt in an sich bekannter Weise, z.B. mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig, wobei auch enzymatische Methoden verwendet werden können.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz, wie oben beschrieben, kann man auch 2-Halogen-niederalkoxycarbonyl (gegebenenfalls nach Umwandlung einer 2-Bromniederalkoxycarbonylgruppe in eine entsprechende 2-Iod-niederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumiodid, gespalten werden kann. Substituiertes 2-Silylethoxycarbonyl kann auch durch Behandeln mit einem, das Fluoridanion liefernden Salz der Fluorwasserstoffsaure, wie einem Alkalimetallfluorid, z.B. Natrium- oder Kaliumfluorid, in Anwesenheit eines macrocyclischen Polyethers ("Kronenether"), oder mit einem Fluorid einer organischen quaternären Base, wie Tetra-niederalkylammoniumfluorid oder Triniederalkyl-aryl-ammoniumfluorid, z.B. Tetraethylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden.

Durch gegebenenfalls substituiertes 1-Phenylniederalkyl, z.B. Benzyl, geschütztes Hydroxy wird vorzugsweise durch katalytische Hydrierung, z.B. in Gegenwart eines Palladium-auf-Kohle-Katalysators freigesetzt. Eine durch 2,2-Dichloracetyl geschützte Hydroxygruppe wird z.B. durch basische Hydrolyse, eine durch tert.-Niederalkyl oder durch einen 2-oxa- oder 2-thia-aliphatischen oder -cycloaliphatischen Kohlenwasserstoffrest veretherte Hydroxygruppe durch Acidolyse, z.B. durch Behandeln mit einer Mineralsäure oder einer starken Carbonsäure, z.B. Trifluoressigsäure, freigesetzt.

Mit einem organischen Silylrest, z.B. Trimethylsilyl, verethertes Hydroxy kann auch mit einem Fluoridanionen liefernden Salz der Fluorwasserstoffsäure, z.B. Tetrabutylammoniumfluorid, freigesetzt werden.

Bevorzugt werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium-Kohle-Katalysator.

Die Herstellung der Ausgangsstoffe der Formel II ist in den europäischen Patentanmeldungen mit den Veröffentlichungsnummern 330 und 114 787 beschrieben.

Verfähren b)

Vorzugsweise verwendet man die Verbindungen der Formel IV in Form ihrer Alkalimetallsalze, z.B. Natriumsalze.

In einer Verbindung der Formel IV in den Resten As und $R^4$ gegebenenfalls vorhandene freie funktionelle Gruppen, die durch eine leicht abspaltbare Schutzgruppe geschützt werden müssen, sind insbesondere freie Hydroxygruppen, aber auch freie Carboxylgruppen. Geeignete Schutzgruppen und ihre Abspaltung sind oben bei Verfahren a) beschrieben.

Ein reaktionsfähiges Carbonsäurederivat einer Verbindung der Formel V, ist in erster Linie ein reaktionsfähiger Ester, ein reaktionsfähiges Anhydrid oder ein reaktionsfähiges cyclisches Amid, worin die Carboxylgruppe in analoger Weise wie bei den bei Verfahren a) beschriebenen reaktionsfähigen Acylierungsmitteln aktiviert ist, und wobei die Aktivierung auch in situ erfolgen kann.

Die Veresterung kann, wenn notwendig, in Gegenwart von geeigneten Kondensationsmitteln, bei Verwendung von freien Carbonsäuren der Formel V z.B. in Gegenwart von Carbodiimidverbindungen, wie Dicyclohexylcarbodiimid, oder Carbonylverbindungen, wie Diimidazolylcarbonyl, und bei Verwendung von reaktionsfähigen Säurederivaten z.B. in Gegenwart von basischen Mitteln, wie Triniederalkylaminen, z.B. Triäthylamin, oder heterocyclischen Basen, z.B. Pyridin oder 4-Dimethylaminopyridin, durchgeführt werden. Die Acylierungsreaktion kann in Abwesenheit oder vorzugsweise in Gegenwart eines Lösungsmittels oder Lösungsmittelgemisches, unter Kühlen, bei Raumtemperatur oder vorzugsweise unter Erwärmen, insbesondere zwischen 20°C und 120°C, und, wenn notwendig, in einem geschlossenen Gefäss und/oder in einer Inertgas-, z.B. Stickstoffatmosphäre, durchgeführt werden. Geeignete Lösungsmittel sind z. B. gegebenenfalls substituierte, insbesondere gegebenenfalls chlorierte, aliphatische, cycloaliphatische oder aromatische Kohlenwasserstoffe, wie Tetrachlorkohlenstoff-Pyridin (1:1), Benzol oder Toluol.

Eine bevorzugte Ausführungsform des Verfahrens b) ist die Veresterung einer Verbindung der Formel IV, worin $Ra^1$ und $Ra^2$ jeweils für Wasserstoff und $Ra^3$ für den obengennanten Rest $R^3$-CO- stehen.

Die Ausgangsstoffe der Formel IV erhält man analog, wie in den europäischen Patentanmeldungen mit den Veröffentlichungsnummern 330 und 114 787 beschrieben ist. Zum Beispiel erhält man ausgehend von (R)-Cystein durch Acylierung mit $R^3$-CO-Cl in Pyridin-Methylenchlorid $R^3$-CO-Cys, welches mit Glyceringlycid in $R^3$-CO-Cys-[2(R,S), 3-dihydroxy-propyl] überführt wird, aus dem man durch Umsetzung mit einer Verbindung der Formel III analog Verfahren a) R3-CO-Cys[2(R,S),3-dihydroxy-propyl]-(As)n-$R^4$ erhält.

Verfahren c)

Eine nucleofuge Gruppe Y ist eine Abgangsgruppe bei einer nucleophilen Substitution, vorzugsweise reaktionsfähiges verestertes Hydroxy, z.B. mit einer starken anorganischen oder organischen Säure verestertes Hydroxy, wie mit einer Mineralsäure, z.B. Halogenwasserstoffsäure, wie Chlorwasserstoff-, Bromwasserstoff- oder Iodwasserstoffsäure, ferner Schwefelsäure, oder Halogenschwefelsäure, z.B. Fluorschwefelsäure, oder mit einer starken organischen Sulfonsäure, wie einer gegebenenfalls, z.B. durch Halogen, wie Fluor, substituierten Niederalkansulfonsäure oder einer aromatischen Sulfonsäure, z.B. einer gegebenenfalls durch Niederalkyl, wie Methyl, Halogen, wie Brom, und/oder Nitro substituierten Benzolsulfonsäure, z.B. einer Methansulfon-, Trifluormethansulfon- oder p-Toluolsulfonsäure, verestertes Hydroxy, bevorzugterweise ein Chlorid, Bromid oder Iodid.

Als Salz einer Verbindung der Formel VII verwendet man vorzugsweise ein Alkalimetallsalz, z.B. ein Natriumsalz.

Funktionelle Gruppen in einer Verbindung der Formel VII, die vorzugsweise durch leicht abspaltbare Schutzgruppen geschützt werden, sind Hydroxy und Carboxy. Geeignete Schutzgruppen und ihre Abspaltung sind oben bei Verfahren a) beschrieben.

Ein reaktionsfähiges Derivat einer Verbindung der Formel VII ist eine Verbindung, in der die Nucleophilie des an der Reaktion teilnehmenden Schwefelatoms erhöht ist, z.B. durch Abspaltung des Protons der Mercaptogruppe. Ein solches reaktionsfähiges Derivat kann gegebenenfalls auch in situ gebildet werden.

Die Reaktion kann z.B. analog durchgeführt werden wie beschrieben in den europäischen Patentanmeldungen mit den Veröffentlichungsnummern 330 und 11 4787. Vorzugsweise wird die Reaktion zwischen etwa -20°C und +120°C, insbesondere zwischen 0°C und +40°C; z.B. bei 0°C, durchgeführt, beispielsweise in absolutem Dimethylformamid in Gegenwart von Diazabicycloundecen und unter Schutzgas.

Die Ausgangsstoffe der Formel VI sind bekannt. Die Ausgangsstoffe der Formel VII erhält man z.B. durch Umsetzung von $R^3$-CO-Cys, dessen Mercaptogruppe vorzugsweise durch eine leicht abspaltbare Schutzgruppe geschützt ist, mit H-(As)n-$R^4$ gemäss Verfahren a) und Abspaltung der Schutzgruppe(n).

Verfähren d)

Ein reaktionsfähiges Derivat einer Verbindung der Formel VIII ist eine Verbindung, in der die Nucleophilie des an der Reaktion teilnehmenden Schwefelatoms erhöht ist, z.B. durch Abspaltung des Protons der Mercaptogruppe. Ein solches reaktionsfähiges Derivat kann gegebenenfalls auch in situ gebildet werden.

Eine nucleofuge Gruppe Y ist z.B. eine der bei Verfahren c) genannten Gruppen.

Funktionelle Gruppen in einer Verbindung der Formel IX, die vorzugsweise in geschützter Form vorliegen, sind Hydroxy- und Carboxygruppen. Geeignete Schutzgruppen und ihre Abspaltung sind oben bei Verfahren a) beschrieben.

Wenn vorstehend nichts anders angegeben, werden die Verfahren a) bis d) in einem inerten Lösungsmittel oder Lösungsmittelgemisch bei einer Temperatur zwischen etwa -20°C und etwa +120°C, und, wenn nötig, unter Schutzgas durchgeführt.

Die Ausgangsstoffe für die oben beschriebenen Verfahren gemäss der vorliegenden Erfindung sind bekannt, z.B. aus den europäischen Patentanmeldungen mit den Veröffentlichungsnummern 330 oder 114 787, oder können in an sich bekannter Weise, z.B. analog den obengenannten Verfahren, hergestellt werden.

Zusatzoperationen: Salze von Verbindungen der Formel I können in an sich bekannter Weise hergestellt werden. So kann man Salze von Verbindungen der Formel I durch Umsetzung mit einer geeigneten Base, z.B. durch Behandeln mit geeigneten Metallverbindungen, wie Alkalimetallsalzen von geeigneten organischen Carbonsäuren, z.B. dem Natriumsalz der α-Ethylcapronsäure, oder mit geeigneten anorganischen Alkali- oder Erdalkalimetallsalzen, insbesondere solchen, die sich von einer schwachen und vorzugsweise flüchtigen Säure ableiten, z.B. Natriumhydrogencarbonat, oder mit Ammoniak oder einem geeigneten organischen Amin bilden. Zur Bildung von Alkalimetallsalzen aus Salzen der Verbindungen der Formel I mit zwei- oder dreiwertigen Ionen, z.B. Calciumionen, kann man vorteilhafterweise die letztgenannten Salze mit einem Alkalimetallsalz eines Komplexbildners umsetzen, welcher eine höhere Bindungsaffinität zu den zwei-oder dreiwertigen Ionen als zu den einwertigen Alkalimetallionen hat, z.B. mit dem Natriumsalz von Ethylendiamintetraessigsäure.

Infolge der engen Beziehung zwischen den Salzen der Verbindungen der Formel I und den freien Säuren beinhaltet im Rahmen dieses Textes sinn- und zweckgemäss die Angabe "Salze" mitunter auch Salz-Säure-Gemische mit einem gewissen Prozentsatz freier Säure, insbesondere bei entsprechend niedrigem pH-Wert des Mediums.

Gemische von Isomeren können in an sich bekannter Weise, z.B. durch fraktionierte Kristallisation, Chromatographie etc. in die einzelnen Isomeren aufgetrennt werden.

Die oben beschriebenen Verfahren, inklusive die Verfahren zur Abspaltung vom Schutzgruppen und die zusätzlichen Verfahrensmassnahmen, werden, wenn nicht anders angegeben, in an sich bekannter Weise, z.B. in An- oder Abwesenheit von Lösungs- und Verdünnungsmitteln, wenn notwendig, in Anwesenheit von Kondensationsmitteln oder

Katalysatoren, bei erniedrigter oder erhöhter Temperatur, z.B. in einem Temperaturbereich von etwa -20°C bis etwa +150°C, insbesondere von etwa +1°C bis etwa +70°C, hauptsächlich zwischen Raumtemperatur (etwa +20°C) und +45°C, in einem geeigneten Gefäss und erforderlichenfalls unter Druck, z.B. dem Eigendruck des Systems, oder in einer Inertgas-, z.B. Stickstoffatmosphäre, durchgeführt.

Dabei sind unter Berücksichtigung aller im Molekül befindlichen Substituenten, wenn erforderlich, z.B. bei Anwesenheit leicht hydrolysierbarer Reste, besonders schonende Reaktionsbedingungen, wie kurze Reaktionszeiten, Verwendung von milden sauren oder basischen Mitteln in niedriger Konzentration, stöchiometrische Mengenverhältnisse, Wahl geeigneter Katalysatoren, Lösungsmittel, Temperaturund/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt oder das Verfahren auf irgendeiner Stufe abbricht oder einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Neue Ausgangsstoffe und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den in dieser Anmeldung als besonders bevorzugt aufgeführten Verbindungen gelangt.

Die Erfindung betrifft auch pharmazeutische Präparate, die eine wirksame Menge, insbesondere eine zur Immunstimulation wirksame Menge, der Aktivsubstanz zusammen mit pharmazeutisch verwendbaren Trägerstoffen enthalten, die sich zur topischen, z.B. auch intranasalen, oder parenteralen, z.B. intravenösen, subcutanen oder intraperitonealen, oder enteralen, z.B. oralen, Verabreichung eignen.

Zur parenteralen Verabreichung eignen sich in erster Linie wässrige Lösungen des Wirkstoffs in wasserlöslicher Form, z.B. in Form eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet, oder wässrige Injektionssuspensionen, welche viskositätserhöhende Stoffe, z.B. Natrium-Carboxymethylcellulose, Sorbit und/oder Dextran und gegebenenfalls auch Stabilisatoren enthalten.

Die pharmazeutischen Präparate für die parenterale Anwendung enthalten in gebrauchsfertiger Form vorzugsweise zwischen 0,1 % und 20 %, insbesondere zwischen 1 % und 10 % des aktiven Stoffes. Nicht gebrauchsfertige Trockenampullen können bis 100 % des aktiven Stoffes enthalten.

Als topisch anwendbare Präparate kommen z.B. Cremes, Salben, Pasten, Schäume, Tinkturen und Lösungen in Betracht, die vorzugsweise von etwa 0,02 % bis etwa 2 % des Wirkstoffs enthalten.

Crèmes sind Oel-in-Wasser-Emulsionen, die mehr als 50 % Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechend nichtionische Emulgatoren, z.B. Fettsäureester von Polyalkoholen oder Aethylenoxidaddukte davon, wie Polyglycerinfettsäureester oder Polyoxyäthylensorbitan-fettsäureester (Tweens), ferner Polyoxyäthylen-fett-alkoholäther oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Cremes vermindern, z.B. Polyalkohole, wie Glycerin, Sorbit, Propylenglykol und/oder Polyäthylenglykole, ferner Konservierungsmittel und Riechstoffe.

Salben sind Wasser-in-Oel-Emulsionen, die bis zu 70 %, vorzugsweise jedoch von etwa 20 % bis etwa 50 % Wasser oder wässerige Phase enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs, enthalten. Emulgatoren sind entsprechende lipophile Substanzen, wie Sorbitan-fettsäureester (Spans), z. B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyäthylenglykol, sowie Konservierungsmittel und Riechstoffe.

Fettsalben sind wasserfrei und enthalten als Grundlage insbesondere Kohlenwasserstoffe, z.B. Paraffin, Vaseline und/oder flüssige Paraffine, ferner natürliche oder partial-synthetische Fette, z.B. Kokosfettsäuretriglycerid, oder vorzugweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmeflüssigkeit steigernden Fettalkohole, Emulgatoren und/oder Zusätze.

Pasten sind Cremes und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z.B. Dichlordifluormethan und Dichlortetrafluoräthan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyäthylensorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel.

Tinkturen und Lösungen weisen meistens eine wässerig-äthanolische Grundlage auf, der u.a. Polyalkohole, z.B. Glycerin, Glykole, und/oder Polyäthylenglykol, als Feuchthaltemittel zur Herabsetzung der Verdunstung, und rückfettende Substanzen, wie Fettsäureester mit niedrigen Polyäthylenglykolen, d.h. im wässrigen Gemisch lösliche, lipophile Substanzen als Ersatz für die der Haut mit dem Aethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung mit einem Teil der Grundlage vermischt und dann dem Rest der Formulierung beigegeben.

Besonders vorteilhaft ist die Verwendung von pharmazeutischen Präparaten in Liposomenform. Das Lipopeptid wird bei der Bildung der Liposomen zugegeben. Die Herstellung von Liposomen und der Einschluss des Wirkstoffs kann auf verschiedene Weise erfolgen und ist in dem Uebersichtsartikel von Kaye, St. B., Cancer Treatment Reviews (1981) 8, 27 - 50, beschrieben. Weitere Verfahren zur Herstellung von Liposomen als Träger von Wirkstoffen sind ebenfalls durch Barenholz et al. in Biochemistry, Vol. 16, No. 12, 2806 - 2810, sowie in den Deutschen Offenlegungsschriften (DOS) 28 19 655, 29 02 672, 25 32 317 und 28 42 608, in der US-Patentschrift 4,053,585 und in der Europäischen Patentanmeldung 36 676 beschrieben.

Man löst beispielsweise die Lipidkomponenten, z.B. Phospholipide, z.B. Phosphatidsäure, Lecithin oder Kephalin, und gegebenenfalls neutrale Lipide, z.B. Cholesterin, zusammen mit dem Lipopeptid in einem organischen Lösungsmittel, z.B. Chloroform/Methanol, auf. Nach dem Eindampfen bleibt eine homogene Filmschicht zurück. Man dispergiert diese in einer wässrigen Phase, z.B. durch Schütteln. Man erhält so multilamellare Liposomen. Bei der anschliessenden Behandlung mit Ultraschall können sich je nach Beschallungsdauer unilamellare Liposomen bilden, die den Wirkstoff enthalten. Die Liposomen-Suspensionen können insbesondere für die parenterale, z.B. subcutane oder intraperitoneale Applikation oder auch topisch, z.B. intranasal, verwendet werden.

Zum Gegenstand der vorliegenden Erfindung gehört insbesondere auch die Verwendung der neuen Lipopeptide gemäss Formel (I) und ihrer genannten Derivate in einem Verfahren zur Stimulation des Immunsystems, wobei die neuen Verbindungen vorzugsweise in Form der oben beschriebenen pharmazeutischen Präparate verabreicht werden.

Die nachfolgenden Beispiele illustrieren die oben beschriebene Erfindung, ohne diese in irgendeinder Form einzuschränken. Temperaturen werden in Celsiusgraden angegeben. RF-Werte werden auf Kieselgel-Dünnschichtplatten (Merck, Darmstadt, Deutschland) ermittelt. Die Zusammensetzung von Lösungsmittelgemischen ist, wenn nicht anders vermerkt, in Volumenteilen angegeben. Die Konzentration, c, der Substanz im Lösungsmittel(gemisch) ist im Falle der optischen Drehung in Prozent (Gewicht/Volumen) angegeben.

Beispiel 1: Man suspendiert 0,344 g (2,75 mMol) 2-Amino-ethansulfonsäure (Taurin) und 0,5 g (2,76 mMol) N-Hydroxy-norbornan-2,3-dicarboximid in 5 ml bidestilliertem Wasser und stellt unter Rühren mit 0,1 N Natronlauge auf pH 7. Die Lösung dampft man im Vakuum zu einem Harz ein, das man in einer Mischung aus 12 ml Dimethylacetamid und 3 ml Wasser löst. Dazu gibt man eine Lösung von 2 g (2,5 mMol) N-Palmitoyl-S-[2(R),3-dilauroyloxypropyl]-(R)-cystein in 5 ml Dimethylacetamid und 0,7 g N,N'-Dicyclohexyl-carbodiimid. Man rührt 18 Stunden bei Raumtemperatur. Dann dampft man im Vakuum zur Trockne und extrahiert den Rückstand zweimal mit je 30 ml Acetonitril und 30 ml Essigsäureethylester bei 50°. Den Rückstand chromatographiert man an 60 g Kieselgel mit Methylenchlorid-Methanol (95:5). Die Hauptfraktion enthält die gewünschte Verbindung mit $R_f$ = 0,35 (Chloroform:Methanol = 9:1).

Zur Entfernung kleiner Mengen Ca-Ionen (0,36 %), die bei der Chromatographie aus dem Kieselgel eluiert werden, wird mit dem Na-Salz von Ethylendiamintetraessigsäure (EDTA) (aus EDTA und 2N Natronlauge bis pH = 7) umgesalzen. 1,1 g des oben gewonnenen Salzgemisches löst man bei 30° in einer Mischung aus 45 ml Dimethoxyethan und 5 ml bidestilliertem Wasser, gibt 60 ml 0,2molare EDTA-Na-Salz-Lösung vom pH = 7 dazu und erhält eine klare Lösung. Man engt im Vakuum auf 20 ml ein und erhält eine Suspension, die in einer Amicon-Rührzelle mit bidestilliertem Wasser über ein Ultrafilter (Ausschlussgrenze 10 000 Dalton; Amicon PM 10) filtriert wird, bis das Filtrat EDTA-negativ ist. Die Substanz bleibt als Gel über dem Filter und wird durch Zugabe von 100 ml Acetonitril ausgefällt und bei 50° im Vakuum getrocknet. Man erhält N-(N-Palmitoyl-S-[2(R),3-dilauroyloxy-propyl]-(R)-cysteinyl)-taurin-nautriumsalz; Smp. ab 270° (Zers.); $[\alpha]_D^{20}$ = -10,3° ± 2° (c = 0,485; Dimethylsulfoxid), $[\alpha]_D^{20}$ = -6,0° ± 1,9° (c = 0,515; Chloroform).

Beispiel 2: Analog Beispiel 1 erhält man aus N,N'-Di-(2-natrium-sulfonatoethyl)-L-glutaminsäurediamid und N-Palmitoyl-S-[2(R),3-dilauroyloxypropyl]-(R)-cystein Palmitoyl-Cys- [2(R),3-dilauroyloxy-propyl]-Glu(NH-$CH_2$-$CH_2$-

$SO_3^{\ominus}Na^{\oplus}$)-NH-$CH_2$-$CH_2$-$SO_3^{\ominus}Na^{\oplus}$ ; Smp. > 250°, ab 290° Zers.; $R_f$ = 0,42 (Chloroform: Methanol:Wasser = 70:30: 3), $[\alpha]_D^{20}$ = + 1,2° ± 1° (c = 0,985; Dimethylsulfoxid).

Das Ausgangsmaterial erhält man auf folgende Weise:

Stufe 2.1:8 8 g N-Benzyloxycarbonyl-L-glutaminsäuredi-(2,4,5-trichlorphenyl)ester, gelöst in 60 ml Dimethylacetamid, versetzt man mit 3,44 g Taurin, gelöst in 13,74 ml 2N Natronlauge. Man lässt 15 Stunden bei Raumtemperatur rühren. Dann stellt man den pH-Wert auf 6 und dampft im Vakuum ein. Man nimmt mit bidestilliertem Wasser auf, saugt Trichlorphenol ab und wäscht mit Wasser nach. Eindampfen der wässrigen Phase liefert ein farbloses Harz, das man je zweimal mit 30 ml Diethylether bei 40°, 30 ml Aceton bei 50° und 30 ml Ethanol bei 60° extrahiert. Man saugt ab und extrahiert den Rückstand zweimal mit 50 ml Methanol.

Eindampfen der Methanolphase liefert die Rohverbindung, die man in 20 ml bidestilliertem Wasser löst und mit 120 ml Aceton ausfällt, worauf man Benzyloxycarbonyl-Glu(NH-$CH_2$-$CH_2$-$SO_3^{\ominus}Na^{\oplus}$)-NH-$CH_2$-$CH_2$-$SO_3^{\ominus}Na^{\oplus}$ erhält; $R_f$ = 0,41 (Chloroform:Methanol:Wasser = 60:40:5), Smp. 229-232° (Zers.).

Stufe 2.2: Aus Benzyloxycarbonyl-Glu(NH-$CH_2$-$CH_2$-$SO_3^{\ominus}Na^{\oplus}$)-NH-$CH_2$-$CH_2$-$SO_3Na^{\oplus}$ erhält man durch katalytische Hydrierung mit Palladium/Kohle (10 % Pd) in Wasser nach dem Gefriertrocknen N,N'-Di-(2-natrium-sulfonato-ethyl)-L-glutaminsäurediamid als amorphes Pulver; $R_f$ = 0,39 (Wasser; ermittelt auf Dünnschichtplatten, die mit Kieselgel UP-$C_{12}$ [mit Dodecylresten beladenes Kieselgel] der Firma Antec beschichtet sind).

Beispiel 3: Man löst 1 g (1,25 mMol) N-Palmitoyl-S-[2(R,S),3-dilauroyloxy-propyl]-(R)-cystein und 203 mg (1,5 mMol) N-Hydroxy-benztriazol in 10 ml absolutem Dimethylacetamid, kühlt auf 0°, gibt 309 mg (1,5 mMol) N,N'-Dicyclohexylcarbodiimid zu und rührt 3 Stunden bei 0°. Dann gibt man eine Lösung von 295 mg (1,5 mMol) L-Alanyltaurin und 220 µl Tetramethylguanidin in 10 ml absolutem Dimethylacetamid zu und rührt 18 Stunden bei Raumtemperatur. Man dampft im Vakuum ein und extrahiert den Rückstand einmal mit 30 ml Essigsäureethylester und einmal mit 30 ml Methylenchlorid. Eindampfen der vom Niederschlag befreiten Lösung liefert das Rohprodukt. Dies löst man in Methylenchlorid und extrahiert dreimal mit gesättigter Natriumhydrogencarbonat-Lösung das N-Hydroxybenztriazol. Die organische Phase wird eingedampft und der Rückstand dreimal mit je 10 ml Acetonitril extrahiert, die Lösungen auf 10° gekühlt und vom Niederschlag abgesaugt. Das in Acetonitril bei 10° unlösliche Material wird mit dem Na-Salz der 2-Ethyl-capronsäure ins Na-Salz verwandelt. Hierzu löst man das Rohprodukt in 10 ml Chloroform und gibt 7 ml einmolare 2-Ethyl-capronsäure-Na-salzlösung in Methanol dazu, dampft zur Trockne, extrahiert den Rückstand dreimal mit je 8 ml Essigsäureethylester und chromatographiert den Rückstand an 30 g mit 6 N Salzsäure extrahiertem Kieselgel (Merck). Man eluiert zuerst mit Methylenchlorid, dann mit Methylenchlorid-Aceton -Aceton (1:1), dann mit Methylenchlorid-Methanol-Wasser (9:1:0,1). Die Substanz wird mit den an letzter und vorletzter Stelle genannten Lösungsmittelgemischen eluiert. Man erhält Palmitoyl-(R,S)-Cys[2(R,S),3-dilauroyloxy-propyl]-Ala-NH-$CH_2$-$CH_2$-$SO_3^{\ominus}Na^{\oplus}$ als farbloses Pulver vom Smp. 228° - 230°; $[\alpha]_D^{20}$ = -3,8° ± 1,1° (c = 0,877; Chloroform:Methanol = 1:1); $R_f$ = 0,196 (Chloroform:Methanol = 1:1). Das als Ausgangsprodukt verwendete L-Alanyl-taurin ist beschrieben im US-Patent 4 666 886, Example 33.

Bemerkungen: Die Chromatographie an unbehandeltem Kieselgel führt zur Elution geringer Mengen Ca-Ionen durch die Substanz. Analog Beispiel 1 können diese mit dem Natriumsalz der Ethylendiamintetraessigsäure ausgetauscht werden. Die physikalischen Eigenschaften wie optische Drehung, Schmelzpunkt und Rf-Wert der $Na^+$- und der gemischten $Na^+/Ca^{++}$-Salze unterscheiden sich nicht. Bei der in Beispiel 3 beschriebenen Kupplungsmethode erfolgt eine Racemisierung am Cystein.

Beispiel 4: Analog Beispiel 3 erhält man aus Palmitoyl-Cys[2(R,S),3-dilauroyloxy-propyl] und Taurin Palmitoyl-(R,S)-Cys[2(R,S),3-dilauroyloxypropyl]-NH-$CH_2$-$CH_2$-$SO_3^{\ominus}Na^{\oplus}$; $R_f$ = 0,29 (Methylenchlorid:Ethanol = 10:1), $[\alpha]_D^{20}$ = -0,6° ± 1,2° (c = 0,865; Chloroform:Methanol = 1:1).

Beispiel 4: Tabletten enthaltend 20 mg an Wirkstoff, z.B. eine der in den vorangehenden Beispielen beschriebenen Verbindungen der Formel I, werden in folgender Zusammensetzung in üblicher Weise hergestellt:

| Zusammensetzung: | |
|---|---|
| Wirkstoff | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung: Der Wirkstoff wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge

Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpresst.

**Patentansprüche**

1. Ein Salz eines Aminosulfonsäurederivats der Formel I,

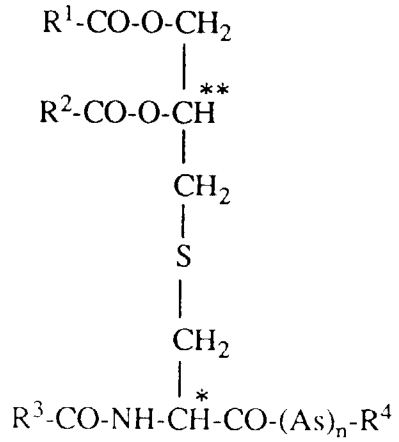

* (R)-Konfiguration

** (R)- oder (S)-Konfiguration

(I)

worin $R^1$, $R^2$ und $R^3$ unabhängig voneinander je einen aliphatischen Kohlenwasserstoffrest mit 7 bis 21 C-Atomen bedeuten, n für 0 oder 1 steht, As den amidisch gebundenen Rest einer (D)- oder (L)-Aminosäure oder eines (D)- oder (L)-Aminosäurederivats aus der Gruppe, bestehend aus Gly, Ala, Ser, Thr, Asp, Asp($R^5$), Glu, Glu($R^5$), Gla, Gla($R^5$) und Gla($R^5$)$_2$ bedeutet und $R^4$ und $R^5$ unabhängig voneinander für den amidisch gebundenen Rest einer unsubstituierten oder durch Carboxy substituierten ω-Amino-$C_2$-$C_3$-alkansulfonsäure stehen.

2. Ein Salz eines Sulfonsäurederivats der Formel I nach Anspruch 1, worin $R^1$, $R^2$ und $R^3$ unabhängig voneinander $C_7$-$C_{21}$-Alkyl, $C_{17}$-Alkenyl oder $C_{17}$-Alkinyl bedeuten, n für 0 oder 1 steht, As den amidisch gebundenen Rest einer Aminosäure oder eines Aminosäurederivats aus der Gruppe, bestehend aus Ala und Glu($R^5$) bedeutet, und $R^4$ und $R^5$ je für den almidisch gebundenen Rest einer ω-Amino-$C_2$ -$C_3$-alkansulfonsäure stehen.

3. Ein Salz eines Sulfonsäurederivats der Formel I nach Anspruch 1, worin $R^1$ und $R^2$ jeweils die gleiche bedeutung haben und jeweils für geradkettiges $C_{11}$-$C_{17}$-Alkyl stehen, $R^3$ geradkettiges $C_{11}$-$C_{17}$-Alkyl bedeutet, n für 0 oder 1 steht, As den amidisch gebundenen Rest von Alanin oder Glu(NH-$CH_2$-$CH_2$-$SO_3H$) bedeutet, und $R^4$ für den amidisch gebundenen Rest von 2-Amino-ethansulfonsäure steht.

4. Ein Salz eines Sulfonsäurederivats der Formel I nach einem der Ansprüche 1 - 3, worin in Formel I $R^1$ für n-Undecyl, $R^2$ für n-Undecyl und $R^3$ für n-Pentadecyl stehen.

5. Ein Salz von N-(N-Palmitoyl-S-[2(R),3-dilauroyloxy-propyl]-(R)-cysteinyl)taurin nach Anspruch 1.

6. Ein Salz von Palmitoyl-Cys[2(R),3-dilauroyloxy-propyl]-Glu(NH-$CH_2$-$CH_2$-$SO_3H$)-NH-$CH_2$-$CH_2$-$SO_3H$ nach Anspruch 1.

7. Ein pharmazeutisch verwendbares Salz einer Verbindung der Formel I nach einem der Ansprüche 1 - 6.

8. Ein pharmazeutisch verwendbares Salz einer Verbindung der Formel I nach einem der Ansprüche 1 - 6 zur Anwendung in einem Verfahren zur therapeutischen Behandlung des menschlichen oder tierischen Körpers.

9. Verwendung eines pharmazeutisch verwendbaren Salzes einer Verbindung der Formel I nach einem der Ansprüche 1 - 6 zur Herstellung von pharmazeutischen Präparaten, die zur Stimulation des Immunsystems von Warmblütern bestimmt sind.

**10.** Pharmmazeutische Präparate, die ein pharmazeutisch verwendbares Salz einer Verbindung der Formel I nach einem der Ansprüche 1-6 zusammen mit pharmazeutischem Trägermaterial enthalten.

**11.** Verfähren zur Herstellung eines Salzes eines Sulfonsäurederivats der Formel I,

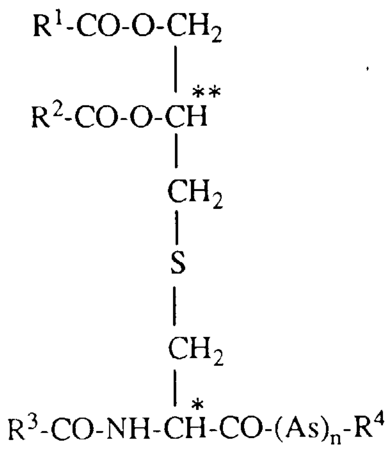

* (R)-Konfiguration

** (R)- oder (S)-Konfiguration

(I)

worin $R^1$, $R^2$ und $R^3$ unabhängig voneinander je einen aliphatischen Kohlenwasserstoffrest mit 7 bis 21 C-Atomen bedeuten, n für 0 oder 1 steht, As den amidisch gebundenen Rest einer (D)- oder (L)-Aminosäure oder eines (D)- oder (L)-Aminosäurederivats aus der Gruppe, bestehend aus Gly, Ala, Ser, Thr, Asp, Asp($R^5$), Glu, Glu($R^5$), Gla, Gla($R^5$) und Gla($R^5$)$_2$ bedeutet und $R^4$ und $R^5$ unabhängig voneinander für den amidisch gebundenen Rest einer unsubstituierten oder durch Carboxy substituierten ω-Amino-$C_2$-$C_3$-alkansulfonsäure stehen, dadurch gekennzeichnet, dass man

a) eine Verbindung der Formel II,

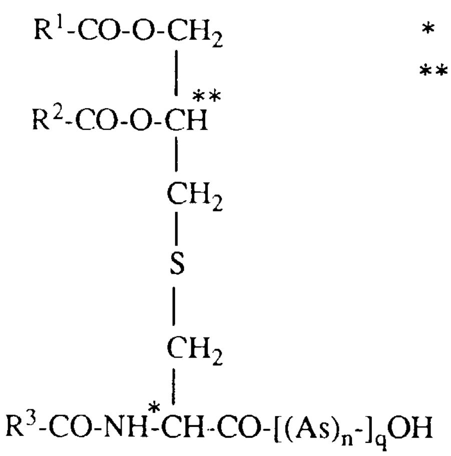

* (R)-Konfiguration

** (R)- oder (S)-Konfiguration

(II)

worin q für 0 oder 1 steht und $R^1$, $R^2$, $R^3$, n und As die obengenannten Bedeutungen haben, wobei im Rest As vorhandene freie funktionelle Gruppen mit Ausnahme der Gruppe, die an der Reaktion teilnehmen soll, erforderlichenfalls durch leicht abspaltbare Schutzgruppen geschützt sind, oder ein relationsfähiges Carbonsäurederivat davon mit dem Salz einer Verbindung der Formel III,

$$H\text{-}[(As)_n\text{-}]_rR^4 \qquad \text{(III)}$$

worin r für 1 steht, wenn im Reaktionspartner der Formel II q für 0 steht, oder r für 0 steht, wenn q für 1 steht, und worin As, n und $R^4$ die obengenannten Bedeutungen haben, wobei in den Resten As und $R^4$ vorhandene freie funktionelle Gruppen mit Ausnahme der Gruppe, die an der Reaktion teilnehmen soll, erforderlichenfalls durch leicht abspaltbare Schutzgruppen geschützt sind, umsetzt und vorhandene Schutzgruppen abspaltet, oder

b) ein Salz einer Verbindung der Formel IV,

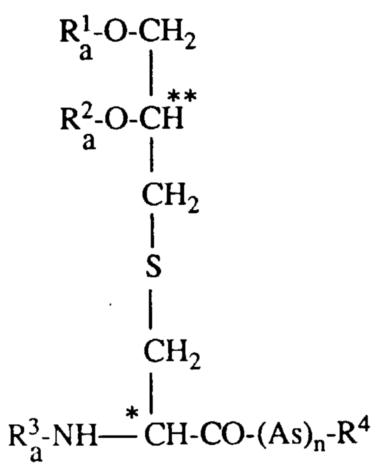

* (R)-Konfiguration

** (R)- oder (S)-Konfiguration

(IV)

worin $R^1a$ für Wasserstoff oder den obengenannten Rest $R^1$-CO-, $R^2a$ für Wasserstoff oder den obengenanten Rest $R^2$-CO- und $R^3a$ für Wasserstoff oder den obengenannten Rest $R^3$-CO- stehen, wobei mindestens einer der Reste $R^1a$, $R^2a$ und $R^3a$ Wasserstoff bedeuten muss, und worin As, n und $R^4$ die obengenannten Bedeutungen haben, wobei in den Resten As und $R^4$ vorhandene freie funktionelle Gruppen mit Ausnahme der Gruppe, die an der Reaktion teilnehmen soll, erforderlichenfalls durch leicht abspaltbare Schutzgruppen geschützt sind, mit einer Carbonsäure der Formel V,

$$R^6\text{-COOH} \qquad \text{(V)}$$

worin $R^6$ einen aliphatischen Kohlenwasserstoffrest mit 7 bis 21 C-Atomen bedeutet, oder mit einem reaktionsfähigen Carbonsäurederivat davon umsetzt und vorhandene Schutzgruppen abspaltet, oder

c) eine Verbindung der Formel VI,

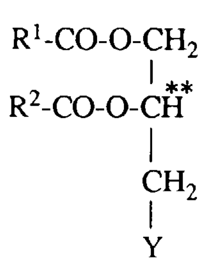

** (R)- or (S)-Konfiguration

(VI)

worin Y für eine nucleofuge Gruppe steht und $R^1$ und $R^2$ die obengenannten Bedeutungen haben, mit dem Salz einer Verbindung der Formel VII,

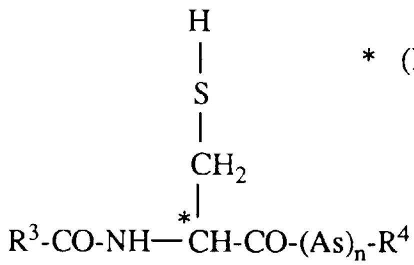

* (R)-Konfiguration

(VII)

worin die Substituenten die obengenannten Bedeutungen haben, wobei freie funktionelle Gruppen mit Ausnahme der an der Reaktion teilnehmenden Mercaptogruppe, wenn nötig, durch leicht abspaltbare Schutzgruppen geschützt sind, oder mit einem reaktionsfähigen Derivat einer Verbindung der Formel VII umsetzt und vorhandene Schutzgruppen abspaltet, oder

(d) eine Verbindung der Formel VIII,

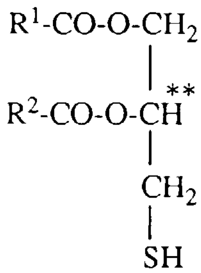

** (R)- or (S)-Konfiguration

(VIII)

worin $R^1$ und $R^2$ die obengenannten Bedeutungen haben, oder ein reaktionsfähiges Derivat dieser Verbindung mit dem Salz einer Verbindung der Formel IX,

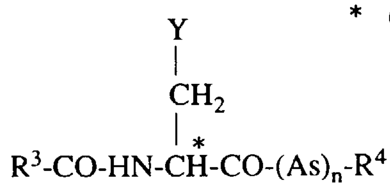

* (R)-Konfiguration

(IX)

worin Y für eine nucleofuge Gruppe steht und die übrigen Substituenten die obengenannten Bedeutungen haben, wobei freie funktionelle Gruppen, wenn nötig, in geschützter Form vorliegen, umsetzt und vorhandene Schutzgruppen abspaltet, und, wenn erwünscht, nach Durchführung einer der Verfahrensvarianten a - d) ein erhaltenes Salz in ein anderes Salz überführt und, wenn erwünscht, erhaltene Isomerengemische auftrennt.

## Claims

**1.** A salt of an aminosulfonic acid derivative of formula I

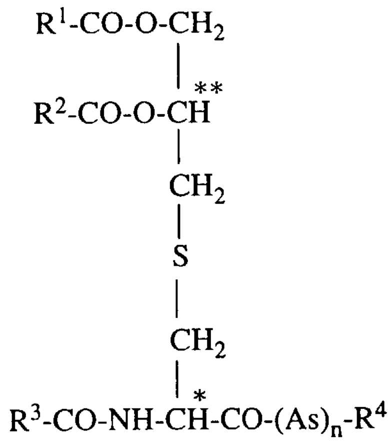

* (R)-configuration

** (R)- or (S)-configuration

(I)

wherein

$R^1$, $R^2$ and $R^3$ are each independently of the others an aliphatic hydrocarbon radical having from 7 to 21 carbon atoms,
n is 0 or 1,
As is the amidically bonded residue of a (D)- or (L)-amino acid or of a (D)- or (L)-amino acid derivative from the group consisting of Gly, Ala, Ser, Thr, Asp, Asp($R^5$), Glu, Glu($R^5$), Gla, Gla($R^5$) and Gla($R^5$)$_2$, and
$R^4$ and $R^5$ are each independently of the other the amidically bonded radical of an unsubstituted or carboxy-substituted ω-amino-$C_2$-$C_3$alkanesulfonic acid.

2. A salt of a sulfonic acid derivative of formula I according to claim 1 wherein

$R^1$, $R^2$ and $R^3$ are each independently of the others $C_7$-$C_{21}$alkyl, $C_{17}$alkenyl or $C_{17}$alkynyl, n is 0 or 1,
As is the amidically bonded residue of an amino acid or of an amino acid derivative from the group consisting of Ala and Glu($R^5$), and
$R^4$ and $R^5$ are each the amidically bonded radical of an ω-amino-$C_2$-$C_3$alkanesulfonic acid.

3. A salt of a sulfonic acid derivative of formula I according to claim 1 wherein

$R^1$ and $R^2$ are identical and are each straight-chain $C_{11}$-$C_{17}$alkyl,
$R^3$ is straight-chain $C_{11}$-$C_{17}$alkyl,
n is 0 or 1,
As is the amidically bonded residue of alanine or Glu(NH-$CH_2$-$CH_2$-$SO_3$H), and
$R^4$ is the amidically bonded radical of 2-aminoethanesulfonic acid.

4. A salt of a sulfonic acid derivative of formula I according to any one of claims 1 to 3 wherein in formula I $R^1$ is n-undecyl, $R^2$ is n-undecyl and $R^3$ is n-pentadecyl.

5. A salt of N-(N-palmitoyl-S-[2(R),3-dilauroyloxy-propyl]-(R)-cysteinyl)-taurine according to claim 1.

6. A salt of palmitoyl-Cys[2(R),3-dilauroyloxy-propyl]-Glu(NH-$CH_2$-$CH_2$-$SO_3$H)-NH-$CH_2$-$CH_2$-$SO_3$H according to claim 1.

7. A pharmaceutically acceptable salt of a compound of formula I according to any one of claims 1 to 6.

8. A pharmaceutically acceptable salt of a compound of formula I according to any one of claims 1 to 6 for use in a method for the therapeutic treatment of the human or animal body.

9. The use of a pharmaceutically acceptable salt of a compound of formula I according to any one of claims 1 to 6 in the preparation of pharmaceutical compositions intended for the stimulation of the immune system of warm-blooded animals.

**10.** A pharmaceutical composition comprising a pharmaceutically acceptable salt of a compound of formula I according to any one of claims 1 to 6 together with a pharmaceutical carrier.

**11.** A process for the preparation of a salt of a sulfonic acid derivative of formula I

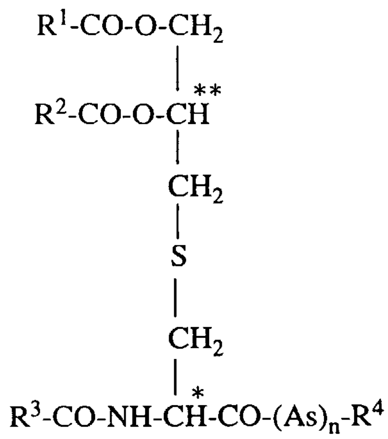

* (R)-configuration
** (R)- or (S)-configuration

(I)

wherein

$R^1$, $R^2$ and $R^3$ are each independently of the others an aliphatic hydrocarbon radical having from 7 to 21 carbon atoms,
n is 0 or 1,
As is the amidically bonded residue of a (D)- or (L)-amino acid or of a (D)- or (L)-amino acid derivative from the group consisting of Gly, Ala, Ser, Thr, Asp, Asp($R^5$), Glu, Glu($R^5$), Gla, Gla($R^5$) and Gla$(R^5)_2$, and
$R^4$ and $R^5$ are each independently of the other the amidically bonded radical of an unsubstituted or carboxy-substituted ω-amino-$C_2$-$C_3$alkanesulfonic acid, which comprises

a) reacting a compound of formula II

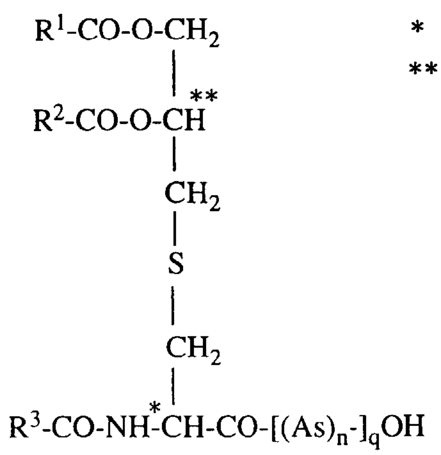

* (R)-configuration
** (R)- or (S)-configuration

(II)

wherein q is 0 or 1 and $R^1$, $R^2$, $R^3$, n and As are as defined above, free functional groups present in the As residue, with the exception of the group that is to participate in the reaction, being protected if necessary by readily removable protecting groups, or a reactive carboxylic acid derivative thereof, with a salt of a compound of formula III

$$H\text{-}[(As)_n\text{-}]_r R^4 \qquad (III)$$

wherein r is 1 if in the co-reactant of formula II q is 0, or r is 0 if q is 1, and wherein As, n and $R^4$ are as defined above, free functional groups present in the radicals As and $R^4$, with the exception of the group that is to participate in the reaction, being protected if necessary by readily removable protecting groups, and removing any protecting groups present, or

b) reacting a salt of a compound of formula IV

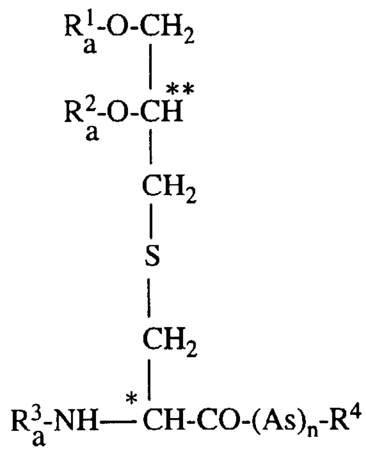

* (R)-configuration
** (R)- or (S)-configuration

(IV)

wherein $R^1{}_a$ is hydrogen or the above-mentioned radical $R^1$-CO-, $R^2{}_a$ is hydrogen or the above-mentioned radical $R^2$-CO- and $R^3{}_a$ is hydrogen or the above-mentioned radical $R^3$-CO-, with the proviso that at least one of the radicals $R^1{}_a$, $R^2{}_a$ and $R^3{}_a$ must be hydrogen, and wherein As, n and $R^4$ are as defined above, free functional groups present in the radicals As and $R^4$, with the exception of the group that is to participate in the reaction, being protected if necessary by readily removable protecting groups, with a carboxylic acid of formula V

$$R^6\text{-COOH} \quad (V),$$

wherein $R^6$ is an aliphatic hydrocarbon radical having from 7 to 21 carbon atoms, or with a reactive carboxylic acid derivative thereof, and removing any protecting groups present, or

c) reacting a compound of formula VI

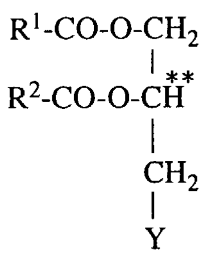

** (R)- or (S)-configuration

(VI)

wherein Y is a nucleofugal group and $R^1$ and $R^2$ are as defined above, with a salt of a compound of formula VII

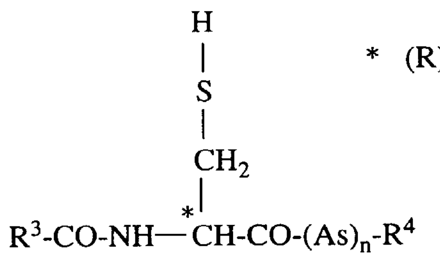

* (R)-configuration

(VII)

wherein the substituents are as defined above, free functional groups, with the exception of the mercapto group that participates in the reaction, being protected if necessary by readily removable protecting groups, or with a reactive derivative of a compound of formula VII, and removing any protecting groups present, or

d) reacting a compound of formula VIII

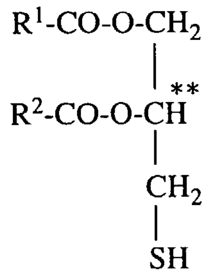

** (R)- or (S)-configuration

(VIII)

wherein $R^1$ and $R^2$ are as defined above, or a reactive derivative of that compound, with a salt of a compound of formula IX

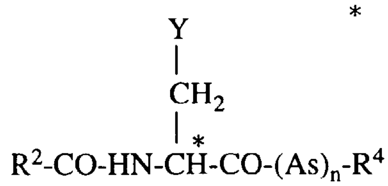

* (R)-configuration

(IX)

wherein Y is a nucleofugal group and the remaining substituents are as defined above, free functional groups being if necessary in protected form, and removing any protecting groups present, and, if desired, after carrying out one of process variants a - d), converting a resulting salt into a different salt and, if desired, separating a resulting mixture of isomers.

**Revendications**

**1.** Un sel d'un dérivé d'acide aminosulfonique de formule I

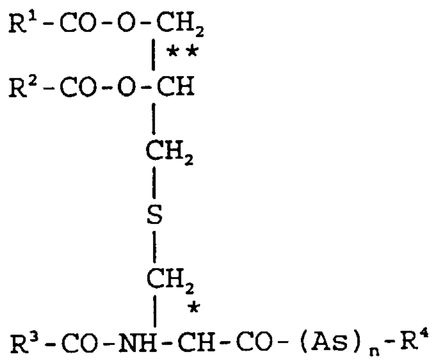

* Configuration (R)
** Configuration (R) ou (S)

(I)

dans laquelle $R^1$, $R^2$ et $R^3$ représentent chacun, indépendamment les uns des autres, un radical hydrocarboné aliphatique en C7-C21, n est égal à 0 ou 1, As représente le radical de liaison amidique d'un (D)- ou (L)-aminoacide ou d'un dérivé de (D)- ou (L)-aminoacide du groupe consistant en Gly, Ala, Ser, Thr, Asp, Asp($R^5$), Glu, Glu($R^5$), Gla, Gla($R^5$) et Gla($R^5$)$_2$, et $R^4$ et $R^5$ représentent chacun, indépendamment l'un de l'autre, le radical à liaison amidique d'un acide ω-amino-(alcane en C2-C3)-sulfonique non substitué ou substitué par des groupes carboxy.

**2.** Un sel d'un dérivé d'acide sulfonique de formule I selon revendication 1, dans laquelle $R^1$, $R^2$ et $R^3$ représentent chacun, indépendamment l'un de l'autre, un groupe alkyle en C7-C21, alcényle en C17 ou alcynyle en C17, n est égal à 0 ou 1, As représente le radical à liaison amiique d'un aminoacide ou d'un dérivé d'aminoacide du groupe consistant en Ala et Glu($R^5$) et $R^4$ et $R^5$ représentent chacun le radical à liaison amidique d'un acide ω-amino-(alcane en C2-C3)-sulfonique.

**3.** Un sel d'un dérivé d'acide sulfonique de formule I selon revendication 1 dans laquelle $R^1$ et $R^2$ ont la même signification et représentent tous deux un groupe alkyle à chaîne droite en C11-C17, $R^3$ représente un groupe alkyle à chaîne droite en C11-C17, n est égal à 0 ou 1, As représente le radical à liaison amidique de l'alanine ou de Glu(NH-$CH_2$-$CH_2$-$SO_3H$) et $R^4$ représente le radical à liaison amidique de l'acide 2-amino-éthanesulfonique.

**4.** Un sel d'un dérivé d'acide sulfonique de formule I selon l'une des revendications 1 à 3, dans laquelle $R^1$ représente un groupe n-undécyle, $R^2$ un groupe n-undécyle et $R^3$ un groupe n-pentadécyle.

**5.** Un sel de la N-(N-palmitoyl-S-[(R),3-dilauroyloxypropyl]-cystéinyl)-taurine selon revendication 1.

**6.** Un sel de palmitoyl-Cys[2(R),3-dilauroyloxypropyl] -Glu (NH-$CH_2$-$CH_2$-$SO_3H$) -NH-$CH_2$-$CH_2$-$SO_3H$ selon revendication 1.

**7.** Un sel acceptable pour l'usage pharmaceutique d'un composé de formule I selon une des revendications 1 à 6.

**8.** Un sel acceptable pour l'usage pharmaceutique d'un composé de formule I selon une des revendications 1 à 6, pour l'utilisation dans un procédé pour le traitement thérapeutique de l'organisme humain ou animal.

**9.** Utilisation d'un sel acceptable pour l'usage pharmaceutique d'un composé de formule I selon une des revendications 1 à 6 pour la préparation de compositions pharmaceutiques conçues pour stimuler le système immunitaire des individus à sang chaud.

**10.** Compositions pharmaceutiques contenant un sel acceptable pour l'usage pharmaceutique d'un composé de formule I selon une des revendications 1 à 6, avec un véhicule pharmaceutique.

**11.** Procédé de préparation d'un sel d'un dérivé d'acide sulfonique de formule I

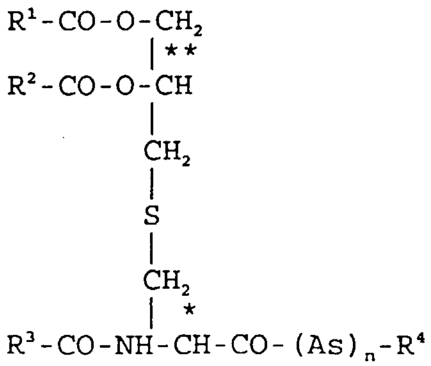

* Configuration (R)
** Configuration (R) ou (S)

(I)

dans laquelle $R^1$, $R^2$ et $R^3$ représentent chacun, indépendamment les uns des autres, un radical hydrocarboné aliphatique en C7-C21, n est égal à 0 ou 1, As représente le radical de liaison amidique d'un (D)- ou (L)-aminoacide ou d'un dérivé de (D)- ou (L)-aminoacide du groupe consistant en Gly, Ala, Ser, Thr, Asp, Asp($R^5$), Glu, Glu($R^5$), Gla, Gla($R^5$) et Gla($R^5$)$_2$, et $R^4$ et $R^5$ représentent chacun, indépendamment l'un de l'autre, le radical à liaison amidique d'un acide ω-amino-(alcane en C2-C3)-sulfonique non substitué ou substitué par des groupes carboxy, caractérisé en ce que

a) on fait réagir un composé de formule II

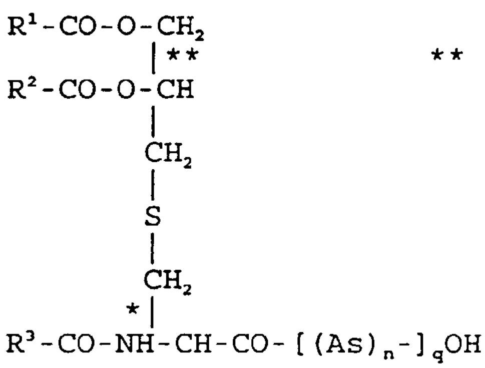

* Configuration (R)
** Configuration (R) ou (S)

(II)

dans laquelle q est égal à 0 ou 1 et $R^1$, $R^2$, $R^3$, n et As ont les significations indiquées ci-dessus, les groupes fonctionnels libres existant dans le radical As, à l'exception du groupe qui doit participer à la réaction, étant protégés lorsque c'est nécessaire par des groupes protecteurs faciles à scinder, ou un dérivé réactif d'acide carboxylique d'un tel composé, avec le sel d'un composé de formule III

$$H\text{-}[(As)_n\text{-}]_r R^4 \qquad \text{(III)}$$

dans laquelle r est égal à 1 lorsque q est égal à 0 dans la formule de l'autre composant, ou à 0 lorsque q est égal à 1, et As, n et $R^4$ ont les significations indiquées ci-dessus, les groupes fonctionnels libres présents dans les radicaux As et $R^4$, à l'exception du groupe qui doit participer à la réaction, étant protégés lorsque c'est nécessaire par des groupes protecteurs faciles à scinder, et on scinde les groupes protecteurs présents, ou bien

b) on fait réagir un sel d'un composé de formule IV,

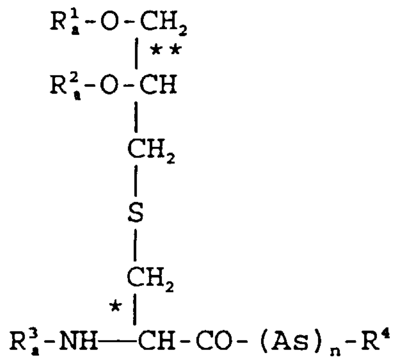

* Configuration (R)
** Configuration (R) ou (S)

(IV)

dans laquelle $R^1a$ représente l'hydrogène ou la radical $R^1$-CO-mentionné ci-dessus, $R^2a$ représente l'hydrogène ou le radical $R^2$-CO- mentionné ci-dessus et $R^3a$ représente l'hydrogène ou le radical $R^3$-CO- mentionné ci-dessus, l'un au moins des symboles $R^1a$, $R^2a$ et $R^3a$ devant représenter l'hydrogène, As, n et $R^4$ ayant les significations indiquées ci-dessus, les groupes fonctionnels libres présents dans les radicaux As et $R^4$, à l'exception du groupe qui doit participer à la réaction, étant protégés lorsque c'est nécessaire par des groupes protecteurs faciles à scinder, avec un acide carboxylique de formule V

$$R^6\text{-COOH} \qquad (V)$$

dans laquelle $R^6$ représente un radical hydrocarboné aliphatique en C7-C21, ou avec un dérivé d'acide carboxylique réactif d'un tel acide carboxylique, et on scinde les groupes protecteurs présents, ou bien
c) on fait réagir un composé de formule VI

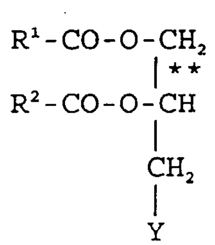

** Configuration (R) ou (S)

(VI)

dans laquelle Y représente un groupe nucléofuge et $R^1$ et $R^2$ ont les significations indiquées ci-dessus, avec le sel d'un composé de formule VII

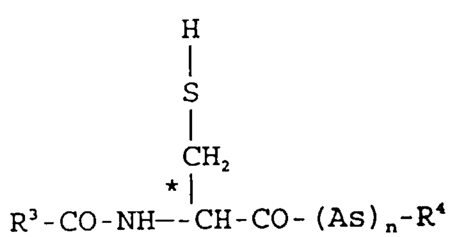

* Configuration (R)

(VII)

dans laquelle les symboles ont les significations indiquées ci-dessus, les groupes fonctionnels libres, à l'exception du groupe mercapto participant à la réaction, étant protégés lorsque c'est nécessaire par des groupes protecteurs faciles à scinder, ou avec un dérivé réactif d'un composé de formule VII, et on scinde les groupes protecteurs présents, ou bien
d) on fait réagir un composé de formule VIII

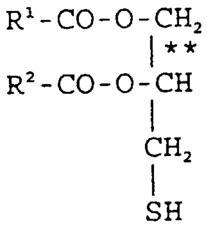

** Configuration (R) ou (S)

(VIII)

dans laquelle $R^1$ et $R^2$ ont les significations indiquées ci-dessus, ou un dérivé réactif de ce composé, avec le sel d'un composé de formule IX

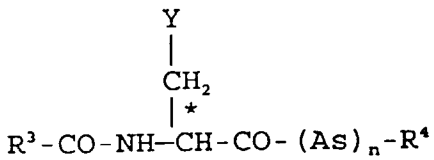

* Configuration (R)

(IX)

dans laquelle Y représente un groupe nucléofuge et les autres symboles ont les significations indiquées ci-dessus, les groupes fonctionnels libres étant protégés lorsque c'est nécessaire, puis on scinde les groupes protecteurs présents et, si on le désire, après exécution de l'une des variantes opératoires a) à d), on convertit un sel obtenu en un autre sel et, si on le désire, on sépare un mélange d'isomères obtenu.